# EUROPÄISCHE PATENTSCHRIFT

(11) **EP 2 100 641 B1**
(45) Veröffentlichungstag und Bekanntmachung des Hinweises auf die Patenterteilung: **02.05.2012**
(21) Anmeldenummer: 09152784.6
(22) Anmeldetag: 13.02.2009
(51) Int. Cl.: A61N 5/10

(54) **Partikeltherapieanlage und Verfahren zur Modulation eines in einem Beschleuniger erzeugten Partikelstrahls**
Particle therapy device and method for modulating a particle beam created in an accelerator
Installation de thérapie à particules et procédé destiné à la modulation d'un rayon de particules produit dans un accélérateur

(30) Priorität: 14.03.2008 DE 102008014406
(43) Veröffentlichungstag der Anmeldung: 16.09.2009
(73) Patentinhaber: Siemens Aktiengesellschaft, 80333 München (DE)
(72) Erfinder: Rietzel, Dr., Eike, 64289 Darmstadt (DE)

(56) Entgegenhaltungen:
- WO-A-02/41948
- WO-A-03/020196
- US-A1- 2001 022 502
- W. T. CHU, B. A. LUDEWIGHT, T. R. RENNER: "Instrumentation for treatment of cancer using proton and light-ion beams" REV. SCI. INSTRUM., Bd. 64, Nr. 8, 1993, XP002530391
- ULI WEBER, GERHARD KRAFT: "Design and construction of a ripple filter for a smoothed depth dose distribution in conformal particle therapy" PHYS. MED. BIOL., Bd. 44, 1999, Seiten 2765-2775, XP002530392

## Beschreibung

Die Erfindung betrifft eine Partikeltherapieanlage mit einem Beschleuniger zum Erzeugen eines Partikelstrahls. Die Erfindung betrifft weiterhin ein Verfahren zur Modulation eines in einem Beschleuniger erzeugten Partikelstrahls.

Bei einer Partikeltherapie, insbesondere von Krebserkrankungen, wird in einem geeigneten Beschleuniger ein Partikelstrahl beispielsweise aus Protonen oder Schwerionen, wie zum Beispiel Kohlenstoffionen, erzeugt. Der Partikelstrahl wird in einer Strahlführung in einen Behandlungsraum geführt und tritt dort über ein Austrittsfenster ein. In einer besonderen Ausführung kann der Partikelstrahl von einem Beschleuniger abwechselnd in verschiedene Behandlungsräume gelenkt werden. In dem Bestrahlungsraum ist ein zu therapierender Patient zum Beispiel auf einem Patiententisch positioniert und gegebenenfalls immobilisiert.

Um eine besonders gute Auslastung der Partikeltherapieanlage, insbesondere des Beschleunigers, zu erreichen, wird der Partikelstrahl nacheinander in unterschiedliche Bestrahlungsräume geführt, um die Zeit vor und nach der Bestrahlung eines Patienten, z.B. die Zeit, die zur Positionierung des Patienten in einem Bestrahlungsraum erforderlich ist, zu nutzen, indem in einem weiteren Bestrahlungsraum ein anderer Patient bestrahlt wird.

Die Bestrahlung des Zielgebiets, gewöhnlich ein Tumor, erfolgt in der Regel schichtweise. In Abhängigkeit von seiner Energie reicht der Partikelstrahl unterschiedlich tief in das Gewebe, so dass das Gewebe sozusagen in scheibenförmige Abschnitte oder Schichten gleicher Eindringtiefe unterteilt werden kann. Der fokussierte Partikelstrahl wird dann über die einzelnen Schichten des Zielgebiets bewegt, was als "beam scanning" bezeichnet wird, so dass innerhalb einer Schicht mehrere Punkte bestrahlt werden, die zum Beispiel auf einem Rastergitter liegen. Indem die Strahlungsintensität bzw. die Energien richtig ausgewählt werden, können auch kompliziert geformte Bereiche genau bestrahlt werden. Die Anordnung der zu bestrahlenden Schichten und Punkte werden so gewählt, dass sich die geplante Dosisverteilung erzielen lässt.

Die beschriebenen Maßnahmen sind bei verschiedenen Scanverfahren verwendbar.

Bei dem so genannten Spotscanverfahren verweist der Partikelstrahl an jedem Zielpunkt für eine vorbestimmte Zeit und/oder deponiert an jedem Zielpunkt eine vorbestimmte Anzahl an Partikel und wird ausgeschaltet, während Ablenkmagnete etc. auf einen nächsten Zielpunkt eingestellt werden.

Bei dem so genannten Rasterscan-Verfahren verweist der Partikelstrahl an jedem Zielpunkt während eine vorbestimmte Zeitdauer oder deponiert an jedem Zielpunkt eine vorbestimmte Anzahl an Partikel, wird aber zwischen den Zielpunkten nicht oder nicht immer ausgeschaltet.

Bei so genannten kontinuierlichen Scanverfahren bilden die Zielpunkte zusammenhängende Linien, bilden also kontinuierliche (oder quasikontinuierliche) Mengen, wobei ihre Anzahl abzählbar unendlich ist. Der Partikelstrahl wird bei einem kontinuierlichen Scanverfahren zumindest innerhalb einer Linie bzw. Zeile in eine Isoenergieschicht kontinuierlich abgelenkt und überstreicht die Zielpunkte, ohne an einzelnen Orten zu verweilen. Mit einer Tiefenmodulationsvorrichtung kann auch ein kontinuierliches Scanverfahren durchgeführt werden, bei dem kontinuierlich die Eindringtiefe des Partikelstrahls moduliert wird.

Die Bewegung des Strahls sowie die Einstellung der Strahlenergie werden von einer Steuereinrichtung angesteuert. Für gewöhnlich wird die Reichweite des Partikelstrahls durch eine Energieeinstellung am Beschleuniger mittels der Steuereinrichtung variiert, sogenannte aktive Energievariation. Die aktive Energievariation bedingt dabei, dass bei im Wesentlichen allen Magneten oder zumindest bei vielen Magneten der nachfolgenden Hochenergiestrahl-Transportstrecke die Magnetstärke auf die Energie des Strahls eingestellt werden muss. Dabei entstehen Wechselzeiten, in denen kein Strahl appliziert werden kann, die Zeit wird aber dennoch für die Bestrahlung benötigt. Die Energiewechselzeit zwischen zwei aufeinanderfolgenden Energieniveaus bzw. Schichten im Zielgebiet, d.h. die Totzeit beim Energiewechsel, beträgt für gewöhnlich etwa 1-2 s. Bei mehreren Energiewechselvorgängen steigt dann die summierte Totzeit auf mehrere Sekunden oder gar auf über eine Minute.

Die US 2001/022502 A1 offenbart eine Partikeltherapieanlage, bei der eine Bestrahlung des Zielvolumens schichtweise erfolgt und die Energie des Partikelstrahls passend zu den Schichten eingestellt wird.

Der Erfindung liegt die Aufgabe zugrunde, eine Optimierung der Bestrahlungszeiten einer Partikeltherapieanlage zu ermöglichen.

Die Aufgabe wird erfindungsgemäß gelöst durch eine Partikeltherapieanlage mit einem Beschleuniger zum Erzeugen eines Partikelstrahls und einem sogenannten passiven Energiemodulator umfassend ein Absorberelement, sowie mit einer Steuereinrichtung, die dafür ausgebildet ist, zur schrittweise Veränderung der Energie des Partikelstrahls von einem hohen auf ein niedriges Energieniveau (oder umgekehrt) zwischen einer aktiven Einstellung der Energie im Beschleuniger und einer passive Energiemodulation, insbesondere durch Energieabsorption, mittels des Energiemodulators umzuschalten.

Die Erfindung geht von der Überlegung aus, dass eine Verbesserung der für die Behandlung eines Patienten benötigten Bestrahlungszeit erreicht ist, indem die Totzeiten zum Wechseln von einem Energieniveau auf das nächste verkürzt werden, weil die passive Energieeinstellung viel schneller durchführbar ist als die aktive. Zwischen dem hohen und dem niedrigen Energieniveau liegen mehrere Energiestufen, die in mehreren Schritten nacheinander eingestellt werden, wobei zwischen je zwei aufeinanderfolgenden Energiestufen ein Energiewechselvorgang erforderlich ist. Den Energiestufen entsprechen beispielsweise unterschiedliche Isoenergieschichten. Um dabei die Totzeiten zu reduzieren, erfolgt der Energiewechsel nicht ausschließlich aktiv über den Beschleuniger, sondern einige der Energiewechselvorgänge werden mittels passiver Absorption mit Hilfe des Energiemodulators durchgeführt. Der Beschleuniger wird dabei weiterhin für eine Energievariation herangezogen, eine Feinabstufung der Energieniveaus - z.B. für die Feinabstufung der Energieniveaus für einzelne Isoenergieschichten - wird jedoch von dem zusätzlichen Energiemodulator übernommen. Während der Schaltzeiten für den Energiemodulator kann der Partikelstrahl kurzzeitig unterbrochen werden. Es ist jedoch auch möglich, insbesondere bei sehr schnellen Schaltzeiten, die Bestrahlung nicht zu unterbrechen. Als Beschleunigereinheit, die eine aktive Einstellung der Energie ermöglicht, kann beispielsweise ein aus mehreren Komponenten zusammengesetzter Beschleuniger eingesetzt werden. Dies kann beispielsweise ein Synchrotron mit einem vorgeschalteten Linearbeschleuniger sein. Der Beschleunigereinheit folgt die Hochenergiestrahl-Transportstrecke. Die aktive Energievariation durch den Beschleuniger bedingt, dass in der nachfolgenden Hochenergiestrahl-Transportstrecke Magnete auf die neue Energie des Partikelstrahls eingestellt werden, was auch entscheidend zu den beschriebenen Totzeiten führt.

Der Energiemodulator umfasst ein Absorberelement, das insbesondere nach Art einer Platte oder eines/mehrerer Keile ausgestaltet ist und im Strahlengang angeordnet ist. Das Material der Platte, z.B. Plexiglas oder Graphit, absorbiert einen Teil der Energie des Partikelstrahls und verändert somit die effektive Reichweite des Strahls im Patienten. Die Reichweitenänderung hängt dabei von der Dicke des Absorberelements in Strahlrichtung ab.

Dank der vorgeschlagenen Ergänzung einer aktiven Energieeinstellung im Beschleuniger durch eine passive Energiemodulation mit Hilfe eines oder mehrerer Energiemodulatoren werden die Vorteile beider Vorgehensweisen kombiniert. Auf der einen Seite werden aufgrund der aktiven Energieeinstellung die Effekte der Sekundärstrahlung klein gehalten. Gleichzeitig wird die Zeit zum Energiewechsel zwischen den Schichten kleiner, da eine Einstellung eines neuen Energieniveaus mit Hilfe des Energiemodulators die Totzeiten deutlich verkürzt. Die verkürzte Behandlungsdauer führt somit bei einer vollen Auslastung des Beschleunigers insbesondere zu einem höheren Patientendurchsatz.

Die Steuereinrichtung ist dafür ausgebildet, zwischen der aktiven Einstellung der Energie des Partikelstrahls und der passiven Energiemodulation mehrmals umzuschalten. Somit erfolgt eine kontrollierte Anwendung beider Energiewechsel-Vorgehensweisen, wobei die Anzahl der Vorgänge zur aktiven Einstellung und die Anzahl der darauf folgenden passiven Energiemodulationen vorgegeben sind. Zum Beginn der Bestrahlung wird mittels des Beschleunigers das hohe Energieniveau eingestellt. Nach einem "beam scanning" der entsprechenden Schicht des zu behandelnden Gewebes wird die Energie des Partikelstrahls um eine Stufe reduziert, um eine weitere Schicht zu bestrahlen. Die Herabsetzung der Energie kann dabei sowohl aktiv als auch passiv erfolgen. Im Hinblick auf eine möglichst große Verkürzung der Totzeiten und unter Berücksichtigung der bei der passiven Energiemodulation entstehenden Sekundärstrahlung wird eine optimale Anzahl von aktiven und passiven Vorgängen zur Energiemodulation ermittelt, um zu bestimmen, welche Vorgänge in einer definierten Reihenfolge appliziert werden.

Die Steuervorrichtung ist dafür eingerichtet, bei jedem zweiten Schritt eine passive Energiemodulation mittels des Energiemodulators vorzunehmen. Hierbei wird jede aktive Energiemodulation im Beschleuniger von einem einzigen Schritt zur passiven Energiemodulation gefolgt. Aufgrund der hohen Anzahl von Schritten zur aktiven Energieeinstellung entsteht sehr wenig Sekundärstrahlung, so dass dieser Betriebsmodus der Partikeltherapieanlage besonders wenig Belastung für die Umgebung bzw. sogar den Patienten ergibt.

Im Hinblick auf eine wesentliche Verringerung der Totzeiten beim Umschalten zwischen zwei benachbarten Energiestufen des Partikelstrahls ist die Steuereinrichtung alternativ dafür eingerichtet, häufiger eine Veränderung der Energie mittels des Energiemodulators als mit Hilfe der aktiven Einstellung im Beschleuniger vorzunehmen, insbesondere in einem Verhältnis von 2:1. Dies bedeutet, dass jeder Schritt der aktiven Energiemodulation von zwei oder mehreren Schritten der passiven Energiemodulation gefolgt ist, bevor die Energie des Partikelstrahls wieder mittels des Beschleunigers variiert wird. Bei der zwei- oder mehrstufigen passiven Modulation der Energie des Partikelstrahls werden insbesondere mehrere Absorberelemente unterschiedlicher Dicke herangezogen, welche automatisch ausgewechselt werden. Um die Energie des Partikelstrahls weiter zu schwächen, sind auch Kombinationen von zwei oder mehreren Absorberelementen möglich.

Vorteilhafterweise sind mehrere Energiemodulatoren an unterschiedlichen Stellen des Strahlengangs angeordnet, insbesondere derart, dass zumindest ein Strahlführungselement zwischen den verschiedenen Energiemodulatoren angeordnet ist. Eine Einstellung der Energie des Partikelstrahls erfolgt dann neben der aktiven Energieeinstellung durch die passive Modulation einer oder mehrerer passiver Energiemodulatoren. Ein Energiemodulator kann z.B. direkt nach dem Beschleuniger angeordnet sein und ein zweiter möglichst nahe an dem Patienten, insbesondere hinter einem Strahlaustrittsfenster in Strahlrichtung. Beispielsweise kann ein passiver Energiemodulator neben einer patientennahen Anordnung auch vor Umlenkmagneten positioniert werden. Dies hat den Vorteil, dass der Partikelstrahl von entstehender Streustrahlung durch den Umlenkmagneten gereinigt wird.

Weiterhin von Vorteil ist, dass für den Energiemodulator mehrere Absorberelemente unterschiedlicher Absorptionsfähigkeit, insbesondere unterschiedlicher Dicke, einzeln oder in Kombination vorgesehen sind. Hierbei sind mehrere Stufen bei der passiven Energiemodulation erzielbar. Es können z.B. Platten mit einer Dicke von 1 mm und 2 mm eingesetzt werden, wobei bei einer Kombination dieser Platten ein Absorptionselement mit einer Dicke von 3 mm gebildet ist. Eine unterschiedliche Absorptionsfähigkeit lässt sich auch durch den Einsatz verschiedener Materialien erreichen.

Vorzugsweise ist der Energiemodulator verschiebbar gelagert. Beispielsweise ist der Energiemodulator zwischen dem Strahlaustrittsfenster und dem Patienten angeordnet und wird möglichst nahe an den Patienten herangefahren, um Streueffekte gering zu halten.

Bevorzugt sind Mittel zur Veränderung der geometrischen Aufweitung des Partikelstrahls vorgesehen. Zur weiteren Beschleunigung der Behandlung eines Patienten wird hierbei zusätzlich zur Verkürzung der Totzeiten bei der Energiemodulation eine Modifikation der räumlichen Energieverteilung des Partikelstrahls vorgenommen, welche zu einer Reduzierung der erforderlichen zu bestrahlenden Schichten führt.

Nach einer bevorzugten Ausgestaltung umfassen die Mittel mindestens einen Ripple-Filter zur Aufweitung der Energieverteilung des Partikelstrahls in Strahlrichtung. Ein Ripple-Filter weist feine Strukturen eines Absorbermaterials auf, wodurch der Partikelstrahl unterschiedlich in der Energie moduliert wird. Die Streuung im Ripple-Filter führt dazu, dass sich die Teilbereiche des Strahls mit unterschiedlicher Energie auf dem Weg zum Zielgebiet wieder vermischen, so dass eine quasi homogene Aufweitung der Energieverteilung erzielt wird. Beim Einsetzen eines Ripple-Filters wird der Partikelstrahl daher derart modifiziert, dass die Kurve, die den Energieverlust über der Eindringtiefe angibt, keinen so stark ausgeprägten Peak aufweist. Stattdessen verlieren die Partikel einen Großteil ihrer Energie bereits bei einer kleineren Eindringtiefe, was durch einen aufgeweiteten Peak graphisch dargestellt ist. Dies entspricht einer Aufweitung der effektiv bestrahlten Schicht. Es sind daher weniger Strahlvorgänge in Strahlrichtung erforderlich, d.h. dank der Aufweitung des Strahles in Strahlrichtung kann die Anzahl der zu bestrahlenden Schichten im Gewebe reduziert werden.

An der Grenze des Tumors zum gesunden Gewebe ist es besonders wichtig, dass so wenig Partikel wie möglich ins gesunde Gewebe eindringen. Daher ist es vorteilhaft, dass im Bereich des hohen und des niedrigen Energieniveaus, welche Niveaus zur Bestrahlung der äußersten, von gesundem Gewebe umgebenden Schichten vorgesehen sind, ein weniger longitudinal aufgeweiteter Strahl eingestellt wird als im mittleren Energiebereich. Vor diesem Hintergrund sind bevorzugt für die unterschiedlichen Energieniveaus unterschiedliche Ripple-Filter vorgesehen. Im Bereich des hohen und des niedrigen Energieniveaus sind z.B. keine Ripple-Filter oder solche Ripple-Filter vorgesehen, welche die Energieverteilung des Partikelstrahls geringfügig beeinflussen, d.h. es wird in diesen Randbereichen ein weniger longitudinal aufgeweiteter Strahl eingestellt, so dass bei der graphischen Visualisierung der Energieverteilung im Strahl ein ausgeprägter Bragg-Peak im Kurvenverlauf gebildet ist. Für die tumorinneren Schichten ist keine klare Abgrenzung zu den benachbarten Schichten erforderlich. Bei der Bestrahlung dieser Schichten können daher Ripple-Filter verwendet werden, die den Strahl deutlich aufweiten, so dass die Anzahl an Bestrahlungsvorgängen in Strahlrichtung wie oben beschrieben verringert wird.

Ein besonders hoher Automatisierungsgrad, der zu einer zusätzlichen Beschleunigung der Bestrahlung führt, wird erreicht, indem die Ripple-Filter für die unterschiedlichen Isoenergieschichten in Strahlrichtung automatisch auswechselbar sind.

Nach einer weiteren bevorzugten Ausgestaltung umfassen die Mittel eine Fokussiervorrichtung zur Veränderung der geometrischen Aufweitung des Partikelstrahls radial zur Strahlrichtung und die Steuervorrichtung ist dafür eingerichtet, einen Rastergitterabstand für den Partikelstrahl zu variieren. Das Rastergitter gibt hierbei die Anzahl und Anordnung der Rasterpunkte innerhalb einer Schicht gleicher Partikelreichweite an, die beim "beam scanning" punktuell mittels des Partikelstrahls bestrahlt werden. Bei der Bestrahlung eines Rasterpunkts wird die Anzahl der Partikel mittels eines Kontrollsystems ermittelt. Wenn die gewünschte Partikelzahl erreicht ist, steuert die Steuereinrichtung den Partikelstrahl so an, dass der nächste Rasterpunkt bestrahlt wird. Mittels der Fokussiervorrichtung wird der Partikelstrahl radial aufgeweitet oder weniger stark fokussiert, so dass die Fläche, auf die die Partikel auftreffen, größer wird. Gleichzeitig werden weniger Rasterpunkte innerhalb der Schicht gebraucht. Somit wird insbesondere die quantitative Kontrolle erleichtert und eine rechtzeitige Reaktion der Steuereinheit beim Erreichen der gewünschten Partikelzahl ermöglicht, da pro Rasterpunkt nun aufgrund der vergrößerten Fläche mehr Partikel benötigt werden und das Kontrollsystem über ausreichende Zeit zum Zählen verfügt, auch wenn schneller bestrahlt wird als mit einem stärker fokussierten Strahl.

Vorteilhafterweise ist die Steuereinrichtung dafür eingerichtet, am Randbereich des Rastergitters einen stärker fokussierten Partikelstrahl einzustellen als im Innenbereich des Rastergitters. Dadurch erfolgen die Anpassungen des Strahlfokus dynamisch, um einen guten Randabfall der Partikeldosis am Rand der Gewebeschicht zu erreichen und somit das angrenzende gesunde Gewebe möglichst gut zu schonen.

Die Aufgabe wird weiterhin erfindungsgemäß gelöst durch ein Verfahren mit den Merkmalen des Anspruchs 8. Therapeutische Verfahren sind nicht Bestandteil der Erfindung. Die im Hinblick auf die Partikeltherapieanlage angeführten Vorteile und bevorzugten Ausgestaltungen sind sinngemäß auf das Verfahren zu übertragen.

So wird die aktive Einstellung der Energie und die passive Energiemodulation mehrmals appliziert. Dabei wird bei jedem zweiten Schritt die passive Energiemodulation angewendet oder die passive Absorption wird häufiger angewendet als die aktive Einstellung.

Ein Ausführungsbeispiel der Erfindung wird im Folgenden anhand einer Zeichnung näher erläutert. Hierin zeigen:
- FIG 1: eine stark vereinfachte schematische Darstellung einer Partikeltherapieanlage und
- FIG 2: in zwei Diagrammen die Energieverteilung mehrerer Partikelstrahlen ohne und mit einem Ripple-Filter zur Aufweitung der Partikelstrahlen in Strahlrich- tung.

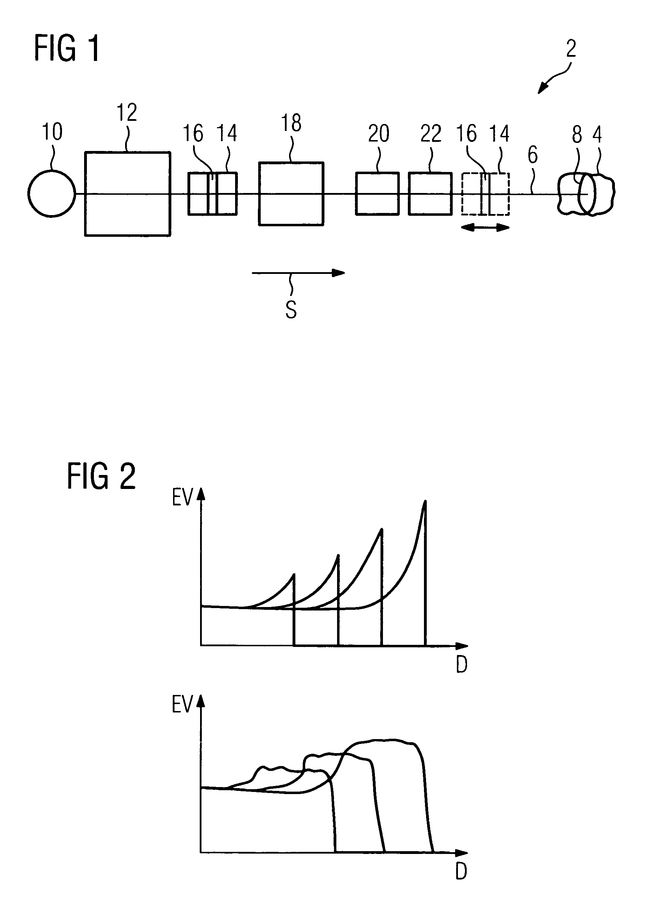

In FIG 1 ist eine Prinzipskizze einer Partikeltherapieanlage 2 gezeigt. Die Partikeltherapieanlage 2 wird eingesetzt, um ein Tumorgewebe 4 mit Hilfe eines Partikelstrahls 6 schrittweise zu bestrahlen, wobei bei jedem Schritt ein scheibenförmiger Abschnitt 8 der Tumors 4, weiterhin auch Schicht genannt, behandelt wird. Der Partikelstrahl 6 wird in einem Beschleuniger 10 generiert, der von einer Steuereinheit 12 angesteuert wird. Der Beschleuniger 10 liefert die Partikel mit einer Energie, die für die aktuell zu bestrahlende Schicht 8 erforderlich ist. Die Steuereinheit 12 umfasst u.a. eine hier nicht gezeigte Rasterscanvorrichtung, die den Strahl 6 sowohl in horizontaler als auch in vertikaler Richtung ablenkt, um das Tumorgewebe 4 innerhalb der Schicht 8 abzutasten. Dazu umfasst die Rasterscanvorrichtung beispielsweise zwei Magnetenpaare.

Der Strahl 6 verläuft außerdem durch einen im Strahlengang angeordneten Energiemodulator 14. Der Energiemodulator 14 umfasst ein Absorberelement 16, welches ein Teil der Energie des Partikelstrahls 6 absorbiert, wenn der Partikelstrahl 6 durch sein Material durchgeht, und somit die Reichweite der Partikel beschränkt. Der Energiemodulator 14 wird somit zur passiven Energiemodulation durch Absorption angewendet. Der Energiemodulator 14 kann dabei innerhalb des gezeigten, schematischen Aufbaus einer Partikeltherapieanlage unterschiedlich angeordnet sein. Er kann sich auch an einer beliebigen anderen Stelle im Vergleich zur dargestellten Reihenfolge der Elemente befinden, wie z.B. direkt vor dem Patienten, was in der Figur durch den gestrichelten Block 14 dargestellt ist. Für eine mögliche Anordnung des passiven Energiemodulationssystems 14 zwischen Strahlaustrittsfenster und Patient kann diese bevorzugt auch möglichst nahe an den Patienten heran verfahren werden, um Streueffekte gering zu halten, was durch einen Pfeil angedeutet ist. Möglich ist auch eine Kombination von zwei oder mehreren Energiemodulatoren 14 an verschiedenen Stellen im Strahlengang.

Anschließend tritt der Partikelstrahl 6 durch ein Kontrollsystem 18, das insbesondere nach Art eines Teilchenzählers ausgebildet ist. Die im Bereich des Tumors 4 deponierte Partikeldosis hängt von der im Strahl 6 vorhandenen Partikelzahl ab. Während des Bestrahlungsvorgangs wird mittels des Kontrollsystems 18 die auf den Tumor 4 einwirkende Partikelzahl ermittelt. Bei Erreichen der gewünschten Partikelzahl in einem Rasterpunkt wird ein Signal an die Steuereinrichtung 12 abgegeben, welche die Rasterscaneinrichtung dafür einsteuert, den Strahl 6 auf den nächsten Rasterpunkt auszurichten.

In dem gezeigten Ausführungsbeispiel sind strahlabwärts außerdem Mittel zur Veränderung der räumlichen Energieverteilung des Partikelstrahls 6 vorgesehen. Die Mittel umfassen hierbei einen Ripple-Filter 20 zur Aufweitung des Strahles 6 in Strahlrichtung S und eine Fokussiervorrichtung 22 zur Aufweitung des Strahls 6 radial zur Strahlrichtung S.

Bei der Bestrahlung des Tumors 4 eines nicht dargestellten Patienten wird über den Beschleuniger 10 ein hohes Energieniveau des Partikelstrahls eingestellt, so dass der Partikelstrahl 8 den in der Figur rechten Randbereich des Tumors 4 erreicht. Dabei werden mehrere Rasterpunkte der erreichten Schicht 8 des Tumors 8 punktuell bestrahlt. Anstelle eines Tumors kann der Partikelstrahl auch auf nicht-lebende Materialien, Zellkulturen oder Phantomen gerichtet werden, wie es beispielsweise im Rahmen von Forschungsarbeiten oder Verifikation von Strahlparametern oftmals praktiziert wird.

Zum Einstellen eines neuen, niedrigeren Energieniveaus zum Bestrahlen einer weiteren Schicht, die sich in diesem Fall links von dem rechten Randbereich im Inneren des Tumors 4 befindet, wird die Energie des Partikelstrahls 6 reduziert. Dies kann auf zwei Weisen erfolgen - entweder mittels einer aktiven Energiemodulation im Beschleuniger 10 oder passiv mit Hilfe des Energiemodulators 16.

Um die bei einem rein aktiven Energiewechsel entstehenden Totzeiten zu verringern, wird bei der in FIG 1 gezeigten Partikeltherapieanlage 2 die aktive Energiemodulation durch eine passive Energieabsorption mittels des Energiemodulators 14 ergänzt, die weniger Zeit zum Einstellen eines niedrigeren Energieniveaus in Anspruch nimmt. Beispielsweise kann ein aktiver Energiewechselvorgang stets durch einen passiven gefolgt werden. Möglich ist auch, dass bei der Einstellung der Energie des Strahls 6 alternierend eine grobe Abstufung mittels des Beschleunigers 10 vorgenommen wird, wobei eine anschließende Feinabstufung in zwei oder mehrere Energiestufen über den Energiemodulator 14 erfolgt. Im vorliegenden Ausführungsbeispiel ist die Steuereinrichtung 12 dafür ausgebildet, nach jeder aktiven Energieeinstellung im Beschleuniger 10 für die zwei darauf folgenden Energiewechsel-Schritte den Energiemodulator 14 heranzuziehen. Für diesen Zweck umfasst der Energiemodulator 14 zwei Absorberelemente 16 unterschiedlicher Dicke, die zum Schichtübergang automatisch ausgewechselt werden. Nach jeder weiteren aktiven Energieeinstellung werden die Absorberelemente in der gleichen Reihenfolge eingesetzt, um zweimal die Energie des Partikelstrahls 6 stufenweise zu reduzieren.

Zur weiteren Beschleunigung des Bestrahlungsvorgangs, wodurch insbesondere ein größerer Patientendurchsatz bei einer hohen Effizienz der Partikeltherapieanlage ermöglicht ist, werden außerdem der Ripple-Filter 20 und die Fokussiervorrichtung 22 angewendet. Die Wirkung des Ripple-Filters 16 wird in FIG 2 näher erläutert. In den Diagrammen gemäß FIG 2 ist jeweils qualitativ der Energieverlust EV gegenüber der Eindringtiefe D des Partikelstrahls 6 im Gewebe aufgetragen. Im oberen Diagramm ist die Energieverteilung für vier zu bestrahlenden Schichten 8 des Tumors 4 gezeigt, wenn kein Ripple-Filter 20 eingesetzt ist. Bei diesem Diagramm sind daher vier Bragg-Peaks deutlich zu erkennen.

Im unteren Diagramm ist die Energieverteilung der Strahlen 6 nach einem Ripple-Filter 20 gezeigt. Aufgrund des Filters 20 ist der Peak jeder der Energieverteilungskurven aufgeweitet oder verbreitet, so dass nach einigen Bestrahlungsvorgängen der tieferen Schichten bereits genug Partikel die vorderen Schichten im Tumor 4 erreicht haben. Aus diesem Grund kann der Tumor 4 in weniger Schichten 8 aufgeteilt werden, die bestrahlt werden sollen, wodurch die Behandlung beschleunigt wird.

Beim Einsatz von Ripple-Filtern 20 kann der Strahl 6 außerdem in den äußersten Schichten im Randbereich des Tumors weniger aufgeweitet sein, damit der Effekt der Bestrahlung auf den Tumor 4 konzentriert ist, ohne das umgebende Gewebe allzu sehr zu belasten. Hierbei können Ripple-Filter 20 verwendet werden, die den Bragg-Peak in der Kurve des Energieverlaufs geringfügig umwandeln oder die Randbereiche können in Abwesenheit von einem Ripple-Filter 20 bestrahlt werden. Bei mehreren Filtern 20 werden diese insbesondere automatisch ausgewechselt.

Um die Dauer der Behandlung weiterhin zu verkürzen, ist im gezeigten Ausführungsbeispiel eine Fokussiervorrichtung 22 vorgehen. Mittels der Fokussiervorrichtung 22 wird die Breite des Strahls 6 eingestellt, d.h. er wird stärker oder weniger stark fokussiert. Zudem ist die Steuereinrichtung 12 derart ausgebildet, dass bei einem weniger stark fokussieren, d.h. bei einem breiteren Strahl 6 weniger Rasterpunkte innerhalb der Schicht 8 bestrahlt werden. Für die Fokussiervorrichtung 22 gilt ebenfalls, dass die Rasterpunkte am Rand der Schicht 8 mit einem stärker fokussieren Strahl 6 bestrahlt werden, damit das umgebende gesunde Gewebe nicht erfasst wird.

## Patentansprüche

1. Partikeltherapieanlage (2) mit einem Beschleuniger (10) zum Erzeugen eines Partikelstrahls (6) und einem passiven Energiemodulator (14) umfassend ein Absorberelement (16), sowie mit einer Steuereinrichtung (12), die dafür ausgebildet ist, zur schrittweise Veränderung der Energie des Partikelstrahls (6) von einem hohen auf ein niedriges Energieniveau zwischen einer aktiven Einstellung der Energie im Beschleuniger (10) und einer passive Energiemodulation mittels des Energiemodulators (14) umzuschalten, wobei zwischen dem hohen Energieniveau und dem niedrigen Energieniveau mehrere Energiestufen liegen, die in mehreren Schritten nacheinander eingestellt werden,
**dadurch gekennzeichnet, dass** die Steuereinrichtung (12) dafür eingerichtet ist, zur Einstellung der mehreren Energiestufen zwischen der aktiven Einstellung der Energie und der passiven Energiemodulation mehrmals umzuschalten, derart, dass bei jedem zweiten Schritt eine passive Energiemodulation mittels des Energiemodulators (14) vorgenommen wird oder dass häufiger eine Veränderung der Energie mittels des Energiemodulators (14) als mit Hilfe der aktiven Einstellung im Beschleuniger (10) vorgenommen wird.

2. Partikeltherapieanlage (2) nach Anspruch 1,
wobei mehrere Energiemodulatoren (14) an verschiedenen Stellen des Strahlengangs angeordnet sind, derart, dass zumindest ein Strahlführungselement zwischen den mehreren Energiemodulatoren angeordnet ist.

3. Partikeltherapieanlage (2) nach einem der vorhergehenden Ansprüche,
wobei Mittel (20,22) zur Veränderung der geometrischen Aufweitung des Partikelstrahls (6) vorgesehen sind.

4. Partikeltherapieanlage (2) nach Anspruch 3,
wobei die Mittel (20, 22) einen Ripple-Filter (20) zur Aufweitung des Partikelstrahls (6) in Strahlrichtung (S) umfassen.

5. Partikeltherapieanlage (2) nach Anspruch 3 oder 4,
wobei für die unterschiedlichen Energieniveaus unterschiedliche Ripple-Filter (20) vorgesehen sind.

6. Partikeltherapieanlage (2) nach einem der Ansprüche 3 bis 5,
wobei die Mittel (20, 22) eine Fokussiervorrichtung (22) zur Veränderung der geometrischen Aufweitung des Partikelstrahls (6) radial zu Strahlrichtung (6) umfassen und die Steuereinrichtung (12) dafür eingerichtet ist, einen Rastergitterabstand für den Partikelstrahl (6) zu variieren.

7. Partikeltherapieanlage (2) nach Anspruch 6,
wobei die Steuereinrichtung (12) dafür eingerichtet ist, am Randbereich des Rastergitters einen stärker fokussierten Partikelstrahl (6) einzustellen als im Innenbereich des Rastergitters.

8. Verfahren zur Modulation eines in einem Beschleuniger (10) erzeugten Partikelstrahls (6),
wobei der Partikelstrahl auf nicht-lebende Materialien, Zellkulturen oder Phantome gerichtet wird,
wobei die Energie des Partikelstrahls (6) schrittweise zwischen einem hohen und einem niedrigen Energieniveau verändert wird, wobei die schrittweise Veränderung durch eine aktive Einstellung der Energie im Beschleuniger (10) und durch eine passive Energiemodulation mit Hilfe eines Energiemodulators (14) im Strahlengang des Partikelstrahls (6) vorgenommen wird, und
wobei zwischen dem hohen Energieniveau und dem niedrigen Energieniveau mehrere Energiestufen liegen, die in mehreren Schritten nacheinander eingestellt werden,
**dadurch gekennzeichnet, dass** zur Einstellung der mehreren Energiestufen zwischen der aktiven Einstellung der Energie und der passiven Energiemodulation mehrmals umgeschaltet wird, derart, dass bei jedem zweiten Schritt eine passive Energiemodulation mittels des Energiemodulators (14) vorgenommen wird oder dass häufiger eine Veränderung der Energie mittels des Energiemodulators (14) als mit Hilfe der aktiven Einstellung im Beschleuniger (10) vorgenommen wird.

9. Verfahren nach Anspruch 8,
wobei die geometrische Aufweitung des Partikelstrahls (6) verändert wird.

10. Verfahren nach Anspruch 9,
wobei die Energie des Partikelstrahls (6) in Strahlrichtung (S) mittels eines Ripple-Filters (22) aufgeweitet wird.

11. Verfahren nach Anspruch 10,
wobei im Bereich des hohen Energieniveaus und des niedrigen Energieniveaus ein weniger longitudinal aufgeweiteter Strahl (6) eingestellt wird als bei den mittleren Energieniveaus.

12. Verfahren nach Anspruch 11,
wobei mehrere Ripple-Filter (20) vorgesehen sind, die automatisch ausgewechselt werden.

13. Verfahren nach einem der Ansprüche 9 bis 12,
wobei die geometrische Aufweitung des Partikelstrahls (6) radial zur Strahlrichtung (s) aufgeweitet wird und ein Rastergitterabstand für den Partikelstrahl (6) variiert wird.

14. Verfahren nach Anspruch 13,
wobei am Randbereich des Rastergitters ein stärker fokussierter Partikelstrahl (6) eingestellt wird als im Innenbereich des Rastergitters.

## Claims

1. Particle therapy apparatus (2) having an accelerator (10) for generating a particle beam (6) and a passive energy modulator (14) comprising an absorber element (16), and having a control entity (12) which, for the purpose of changing the energy of the particle beam (6) from a high energy level to a low energy level in a step-by-step manner, is designed to switch between an active adjustment of the energy in the accelerator (10) and a passive energy modulation by means of the energy modulator (14), wherein between the high energy level and the low energy level are a plurality of energy stages which are successively adjusted in a plurality of steps,
**characterised in that**
the control entity (12) is configured to switch between the active adjustment of the energy and the passive energy modulation multiple times in order to adjust the plurality of energy stages such that a passive energy modulation by means of the energy modulator (14) is effected at each second step or that a change of the energy by means of the energy modulator (14) is effected more frequently than with the aid of the active adjustment in the accelerator (10).

2. Particle therapy apparatus (2) according to claim 1,
wherein a plurality of energy modulators (14) are arranged at different positions of the beam path, such that at least one beam guide element is arranged between the plurality of energy modulators.

3. Particle therapy apparatus (2) according to one of the preceding claims,
wherein means (20,22) are provided for changing the geometric expansion of the particle beam (6).

4. Particle therapy apparatus (2) according to claim 3,
wherein the means (20,22) comprise a ripple filter (20) for expanding the particle beam (6) in the beam direction (S).

5. Particle therapy apparatus (2) according to claim 3 or 4,
wherein different ripple filters (20) are provided for the different energy levels.

6. Particle therapy apparatus (2) according to one of the claims 3 to 5,
wherein the means (20,22) comprise a focusing device (22) for changing the geometric expansion of the particle beam (6) radially relative to the beam direction (6), and the control entity (12) is configured to vary a raster grid distance for the particle beam (6).

7. Particle therapy apparatus (2) according to claim 6,
wherein the control entity (12) is configured to set a more sharply focused particle beam (6) at the peripheral region of the raster grid than in the inner region of the raster grid.

8. Method for modulating a particle beam (6) which is generated in an accelerator (10),
wherein the particle beam is directed at non-living materials, cell cultures or phantoms,
wherein the energy of the particle beam (6) is changed in a step-by-step manner between a high energy level and a low energy level, wherein the step-by-step change is effected by means of an active adjustment of the energy in the accelerator (10) and by means of a passive energy modulation using an energy modulator (14) in the beam path of the particle beam (6), and
wherein between the high energy level and the low energy level are a plurality of energy stages which are successively adjusted in a plurality of steps,
**characterised in that**
a switch is effected between the active adjustment of the energy and the passive energy modulation multiple times in order to adjust the plurality of energy stages such that a passive energy modulation by means of the energy modulator (14) is effected at each second step or that a change of the energy by means of the energy modulator (14) is effected more frequently than with the aid of the active adjustment in the accelerator (10).

9. Method according to claim 8,
wherein the geometric expansion of the particle beam (6) is changed.

10. Method according to claim 9,
wherein the energy of the particle beam (6) in the beam direction (S) is expanded by means of a ripple filter (22).

11. Method according to claim 10,
wherein a less longitudinally expanded beam (6) is set in the region of the high energy level and the low energy level than is set in the case of the midrange energy level.

12. Method according to claim 11,
wherein provision is made for a plurality of ripple filters (20) which are exchanged automatically.

13. Method according to one of the claims 9 to 12,
wherein the geometric expansion of the particle beam (6) is expanded radially relative to the beam direction (S) and a raster grid distance for the particle beam (6) is varied.

14. Method according to claim 13,
wherein a more sharply focused particle beam (6) is set at the peripheral region of the raster grid than in the inner region of the raster grid.

## Revendications

1. Installation de thérapie à particules (2) avec un accélérateur (10) pour générer un rayon de particules (6) et avec un modulateur d'énergie passif (14) comprenant un élément absorbeur (16), ainsi qu'avec un dispositif de commande (12) qui est réalisé pour commuter entre un réglage actif de l'énergie dans l'accélérateur (10) et une modulation passive de l'énergie au moyen du modulateur d'énergie (14) afin de modifier progressivement l'énergie du rayon de particules (6) pour la faire passer d'un niveau d'énergie haut à un niveau d'énergie bas, entre le niveau d'énergie haut et le niveau d'énergie bas se trouvant plusieurs niveaux d'énergie qui sont réglés successivement en plusieurs étapes, **caractérisée en ce que** le dispositif de commande (12) est aménagé pour commuter plusieurs fois, aux fins du réglage des plusieurs niveaux d'énergie, entre le réglage actif de l'énergie et la modulation passive de l'énergie de manière telle que, lors d'une étape sur deux, une modulation d'énergie passive est effectuée au moyen du modulateur d'énergie (14) ou qu'une modification de l'énergie est effectuée plus souvent au moyen du modulateur d'énergie (14) qu'à l'aide du réglage actif dans l'accélérateur (10).

2. Installation de thérapie à particules (2) selon la revendication 1, plusieurs modulateurs d'énergie (14) étant disposés à différents endroits de la trajectoire du rayon de manière telle qu'au moins un élément de guidage de rayon se trouve entre les plusieurs modulateurs d'énergie.

3. Installation de thérapie à particules (2) selon l'une des revendications précédentes, des moyens (20, 22) pour modifier l'élargissement géométrique du rayon de particules (6) étant prévus.

4. Installation de thérapie à particules (2) selon la revendication 3, les moyens (20, 22) comprenant un filtre d'ondulation (20) pour élargir le rayon de particules (6) dans le sense du rayon (S).

5. Installation de thérapie à particules (2) selon la revendication 3 ou 4, différents filtres d'ondulation (20) étant prévus pour les différents niveaux d'énergie.

6. Installation de thérapie à particules (2) selon l'une des revendications 3 à 5, les moyens (20, 22) comprenant un dispositif de focalisation (22) pour modifier l'élargissement géométrique du rayon de particules (6) radialement par rapport au sens du rayon (6) et le dispositif de commande (12) étant aménagé pour faire varier une distance de quadrillage pour le rayon de particules (6).

7. Installation de thérapie à particules (2) selon la revendication 6, le dispositif de commande (12) étant aménagé pour régler au niveau de la zone de bord du quadrillage un rayon de particules (6) focalisé plus fortement que dans la partie intérieure du quadrillage.

8. Procédé de modulation d'un rayon de particules (6) généré dans un accélérateur (10),
le rayon de particules étant dirigé sur des matières non vivantes, des cultures de cellules ou des fantômes, l'énergie du rayon de particules (6) étant modifiée progressivement entre un niveau d'énergie haut et un niveau d'énergie bas, la modification progressive étant effectuée par un réglage actif de l'énergie dans l'accélérateur (10) et par une modulation passive de l'énergie à l'aide d'un modulateur d'énergie (14) dans la trajectoire du rayon de particules (6) et
plusieurs niveaux d'énergie qui sont réglés successivement en plusieurs étapes étant réglés entre le niveau d'énergie haut et le niveau d'énergie bas, **caractérisé en ce que**
il se produit plusieurs commutations, aux fins du réglage des plusieurs niveaux d'énergie, entre le réglage actif de l'énergie et la modulation passive de l'énergie de manière telle que, lors d'une étape sur deux, une modulation d'énergie passive est effectuée au moyen du modulateur d'énergie (14) ou qu'une modification de l'énergie est effectuée plus souvent au moyen du modulateur d'énergie (14) qu'à l'aide du réglage actif dans l'accélérateur (10).

9. Procédé selon la revendication 8, l'élargissement géométrique du rayon de particules (6) se voyant modifié.

10. Procédé selon la revendication 9, l'énergie du rayon de particules (6) étant élargie dans le sense du rayon (S) au moyen d'un filtre d'ondulation (22).

11. Procédé selon la revendication 10, dans la zone du niveau d'énergie haut et du niveau d'énergie bas étant réglé un rayon longitudinalement moins élargi (6) que pour les niveaux d'énergie moyens.

12. Procédé selon la revendication 11, plusieurs filtres d'ondulation (20) étant prévus, lesquels sont remplacés automatiquement.

13. Procédé selon l'une des revendications 9 à 12, l'élargissement géométrique du rayon de particules (6) subissant un élargissement radial par rapport au sens du rayon (s) et une distance de quadrillage pour le rayon de particules (6) subissant des variations.

14. Procédé selon la revendication 13, dans la zone de bord du quadrillage étant réglé un rayon de particules (6) plus fortement focalisé que dans la zone intérieure du quadrillage.
